# EUROPEAN PATENT APPLICATION

(11) **EP 2 305 834 A1**
(43) Date of publication of application: **06.04.2011**
(21) Application number: 09012527.9
(22) Date of filing: 02.10.2009
(51) Int. Cl.: C12Q 1/68

(54) **Haplotype of KATIII gene**

(71) Applicant: Advanced Practical Diagnostics N.V., 2300 Turnhout (BE); Katholieke Universiteit Leuven, K.U. Leuven R&D, 3000 Leuven (BE); Westfälische Wilhelms-Universität Münster, 48149 Münster (DE)
(72) Inventor: Myint, Aye Mu, 2300 Turnhout (BE); Claes, Stephan, 3000 Leuven (BE); Rothermundt, Matthias, 48149 Münster (DE)
(74) Representative: Müller-Boré & Partner Patentanwälte

(57) **Abstract**

The present invention relates to a diagnostic marker comprising at least one single nucleotide polymorphism (SNP) in the human KATIII gene, as well as to a method for the detection of a predisposition for a psychiatric disorder or a psychiatric disease.

## Description

The present invention relates to a diagnostic marker comprising at least one single nucleotide polymorphism (SNP) in the human KATIII gene, as well as to a method for the detection of a predisposition for a psychiatric disorder or a psychiatric disease.

Major depressive disorder (MDD) is a psychiatric disorder with complex etiology. A large twin study showed a moderate heritability of 42% in women and 29% in men. Many genetic association studies have been undertaken, but results have often been difficult to replicate. Meta-analyses have yielded inconsistent results as well. As in other complex moderately heritable disorders, genome-wide association studies might help to overcome these difficulties. However, the first published results of such studies did not yield clear-cut evidence.

In addition to such systematic approaches, hypothesis-driven approaches have been taken, where genes involved in particular relevant pathways were examined in more detail. Regarding the genetic vulnerability for MDD, a lot of attention has been going to serotonin pathways, to other monoaminergic systems and to the hypothalamic-pituitary-adrenal (HPA) axis.

A consistent finding in MDD research is dysfunction of the autoimmune system. Immune activation was well documented in patients with MDD. One of the relevant metabolic pathways in this regard is the kynurenine pathway which forms a bridge between immune activation, serotonergic deficiency and disturbed NMDA-R function.

The kynurenine pathway is part of the tryptophan breakdown pathway. Tryptophan is degraded to kynurenine by the tryptophan 2,3-dioxygenase

(TDO) in the liver and the indoleamine 2,3-dioxygenase (IDO) in the lungs, placenta, blood and the brain. IDO activity is enhanced by pro-inflammatory cytokines such as interferon and inhibited by the anti-inflammatory cytokine IL-4. After tryptophan is first catabolized into kynurenine, kynurenine is further catabolized into 3-hydroxy kynurenine (30HK) by kynurenine-3-monooxygenase (K3MO) and then to the NMDA receptor agonist quinolinic acid. These metabolites contribute to the neuroprotective-neurodegenerative changes in the brain. The activity of K3MO is also enhanced by pro-inflammatory cytokines. 3OHK causes neuronal apoptosis, while quinolinic acid causes excitotoxic neurodegenerative changes. However, kynurenine can also be catabolized by the kynurenine aminotransferases (KATs) into kynurenic acid, which is a NMDA receptor antagonist and is protective against excitotoxic action of quinolinic acid. In patients with major depression, an imbalance between those neuroprotective and neurotoxic pathways has been proposed and demonstrated.

However, the search for genes contributing to MDD vulnerability remains difficult. Therefore, the technical problem underlying the present invention is to provide a novel genetic diagnostic marker being indicative of a predisposition for MDD and related psychiatric disorders and/or diseases.

The solution to the above technical problem is achieved by the embodiments characterized in the claims.

In particular, the present invention relates to a diagnostic marker comprising at least one single nucleotide polymorphism (SNP) in the human KATIII gene.

The term "single nucleotide polymorphism (SNP)" as used herein relates to a DNA sequence variation occurring when a single nucleotide in the sequence differs between members of a species. In particular, a single nucleotide may be substituted, deleted or inserted in the sequence.

In a preferred embodiment of the present invention, the diagnostic marker comprises the SNP rs12729558. In a further preferred embodiment of the present invention, the diagnostic marker further comprises at least one further SNP selected from the group consisting of SNPs rs17130657, rs1325924, rs2038905, rs7556189, rs7517036 and combinations thereof. In a particularly preferred embodiment of the present invention, the diagnostic marker consists of the SNPs rs12729558, rs17130657, rs1325924, rs2038905, rs7556189, and rs7517036. The designation of SNPs as used herein refers to Genbank Database SNP IDs (Nucleic Acids Research, 2008 Jan; 36 (Database issue): D25-30).

In another preferred embodiment of the present invention, the diagnostic marker is indicative of a predisposition for a psychiatric disorder or a psychiatric disease. In a particularly preferred embodiment of the present invention,
the haplotype C in SNP rs12729558; or
the haplotype C in SNP rs12729558 and the haplotype G in SNP rs17130657; or
the haplotype C in SNP rs12729558 and the haplotype G in SNP rs17130657 and the haplotype C in SNP rs1325924;
or the haplotype C in SNP rs12729558 and the haplotype G in SNP rs17130657 and the haplotype C in SNP rs1325924 and the haplotype T in SNP rs2038905; or
the haplotype C in SNP rs12729558 and the haplotype G in SNP rs17130657 and the haplotype C in SNP rs1325924 and the haplotype T in SNP rs2038905 and the haplotype C in SNP rs7556189; or
the haplotype C in SNP rs12729558 and the haplotype G in SNP rs17130657 and the haplotype C in SNP rs1325924 and the haplotype T in SNP rs2038905 and the haplotype C in SNP rs7556189 and the haplotype T in SNP rs7517036; indicates a predisposition for a psychiatric disorder or a psychiatric disease.

In a preferred embodiment, the psychiatric disorder or psychiatric disease for which the diagnostic marker of the present invention is indicative is selected from the group consisting of mood disorders, bipolar disorder, major depressive episode (MDE), major depressive disorder (MDD), panic disorder and anxiety disorders. In a particularly preferred embodiment, the psychiatric disorder or psychiatric disease is MDD.

In a preferred embodiment, the presence of at least one of the diagnostic markers of the present invention indicates an at least 2-fold increased risk, preferably at least 3-fold increased risk for MDE and/or an at least 2-fold increased risk, preferably at least 2.7-fold increased risk for MDD and/or an at least 3-fold increased risk, preferably at least 3.7-fold increased risk for bipolar disorder.

In another aspect, the present invention relates to a method for the detection of a predisposition for a psychiatric disorder or a psychiatric disease in a human patient, comprising the steps of:
(a) providing a genetic sample derived from the patient; and
(b) determining the haplotype of at least one single nucleotide polymorphism (SNP) in the patient's KATIII gene *in vitro.*

Methods for obtaining a genetic sample from a patient are not particularly limited and are well known to a person skilled in the art. In particular, genetic samples may be obtained e.g. by taking a tissue or blood sample from the patient.

Methods for determining the haplotype of one or more SNPs in a particular gene are not particularly limited and are well known to a person skilled in the art. For example, the gene, optionally including DNA regions upstream and/or downstream of the coding region, may be sequenced by PCR-based methods well known in the art. A particular example for such a method is the MassARRAY iPLEX Gold® technology from Sequenom. Primers for use in such PCR-based methods are well known in the art and may be designed by methods well known in the art, e.g. by using the Assay Design program (Sequenom). Methods for SNP analysis and scoring are not particularly limited and are well known in the art. A particular example of a software package suitable for SNP allele calling and analysis is Typer (Sequenom).

In a preferred embodiment of the method of the present invention, the at least one SNP comprises the SNP rs12729558. In another preferred embodiment of the method of the present invention, further the haplotype of at least one further SNP, selected from the group consisting of SNPs rs17130657, rs1325924, rs2038905, rs7556189, rs7517036 and combinations thereof is determined. Accordingly, in a preferred embodiment the haplotype of a combination of SNP rs12729558 with at least one SNP selected from the group consisting of SNPs rs17130657, rs1325924, rs2038905, rs7556189, rs7517036 and combinations thereof is determined. In a, particularly preferred embodiment of the method of the present invention, the haplotype of SNPs rs12729558, rs17130657, rs1325924, rs2038905, rs7556189, and rs7517036 is determined.

In another preferred embodiment, the method of the present invention further comprises the step of correlating
the haplotype C in SNP rs12729558; or
the haplotype C in SNP rs12729558 and the haplotype G in SNP rs17130657; or
the haplotype C in SNP rs12729558 and the haplotype G in SNP rs17130657 and the haplotype C in SNP rs1325924;
or the haplotype C in SNP rs12729558 and the haplotype G in SNP rs17130657 and the haplotype C in SNP rs1325924 and the haplotype T in SNP rs2038905; or
the haplotype C in SNP rs12729558 and the haplotype G in SNP rs17130657 and the haplotype C in SNP rs1325924 and the haplotype T in SNP rs2038905 and the haplotype C in SNP rs7556189; or
the haplotype C in SNP rs12729558 and the haplotype G in SNP rs17130657 and the haplotype C in SNP rs1325924 and the haplotype T in SNP rs2038905 and the haplotype C in SNP rs7556189 and the haplotype T in SNP rs7517036; to a predisposition for a psychiatric disorder or a psychiatric disease.

In a preferred embodiment of the method of the present invention, the psychiatric disorder or psychiatric disease is selected from the group consisting of mood disorders, bipolar disorder, major depressive episode (MDE), major depressive disorder (MDD), panic disorder and anxiety disorders. In a particularly preferred embodiment of the method of the present invention, the psychiatric disorder or psychiatric disease is MDD.

In another aspect, the present invention relates to the use of at least one single nucleotide polymorphism (SNP) in the human KATIII gene as a diagnostic marker. In a preferred embodiment, the diagnostic marker is indicative of a predisposition for a psychiatric disorder or a psychiatric disease.

In a preferred embodiment of the use of the present invention, the diagnostic marker comprises the SNP rs12729558. In a further preferred embodiment of the use of the present invention, the diagnostic marker further comprises at least one further SNP selected from the group consisting of SNPs rs17130657, rs1325924, rs2038905, rs7556189, rs7517036 and combinations thereof. In a particularly preferred embodiment of the use of the present invention, the diagnostic marker consists of the SNPs rs12729558, rs17130657, rs1325924, rs2038905, rs7556189, and rs7517036.

In another preferred embodiment of the use of the present invention,
the haplotype C in SNP rs12729558; or
the haplotype C in SNP rs12729558 and the haplotype G in SNP rs17130657; or
the haplotype C in SNP rs12729558 and the haplotype G in SNP rs17130657 and the haplotype C in SNP rs1325924;
or the haplotype C in SNP rs12729558 and the haplotype G in SNP rs17130657 and the haplotype C in SNP rs1325924 and the haplotype T in SNP rs2038905; or
the haplotype C in SNP rs12729558 and the haplotype G in SNP rs17130657 and the haplotype C in SNP rs1325924 and the haplotype T in SNP rs2038905 and the haplotype C in SNP rs7556189; or
the haplotype C in SNP rs12729558 and the haplotype G in SNP rs17130657 and the haplotype C in SNP rs1325924 and the haplotype T in SNP rs2038905 and the haplotype C in SNP rs7556189 and the haplotype T in SNP rs7517036; indicates a predisposition for a psychiatric disorder or a psychiatric disease.

In a preferred embodiment of the use of the present invention, the psychiatric disorder or psychiatric disease is selected from the group consisting of mood disorders, bipolar disorder, major depressive episode (MDE), major depressive disorder (MDD), panic disorder and anxiety disorders. In a particularly preferred embodiment of the use of the present invention, the psychiatric disorder or psychiatric disease is MDD.

In preferred embodiments of the use of the present invention, the at least one SNP in the human KATIII gene and the diagnostic marker are as defined above.

In another aspect, the present invention relates to at least on single nucleotide polymorphism (SNP) in the human KATIII gene for use as a diagnostic marker.

In preferred embodiments of this aspect of the present invention, the at least one SNP in the human KATIII gene and the diagnostic marker are as defined above.

In yet another aspect, the present invention relates to a kit for use in the method of the present invention. In particular, the kit of the present invention comprises means for carrying out the method of the present invention. Means for carrying out the method of the present invention include, but are not limited to, suitable buffers, reagents and consumables.

The figure shows:

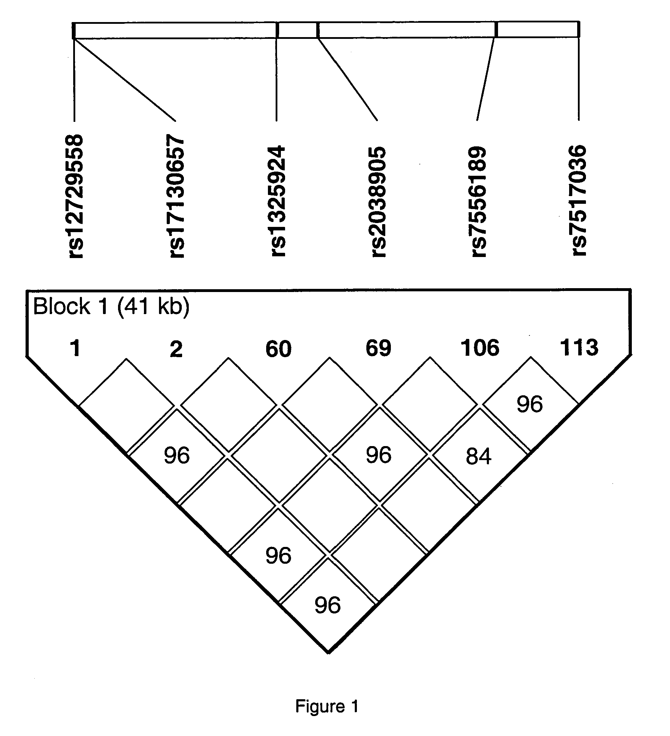

Figure 1: KATIII linkage disequilibrium (LD) structure

The linkage disequilibrium (LD) structure of KATIII consists of one single haplotype block.

The present invention will now be further illustrated in the following examples without being limited thereto.

### Examples

### Experimental strategy:

Given the potential importance of the kynurenine pathway in the pathogenesis of major depressive disorder (MDD), a single nucleotide polymorphism (SNP) based gene association analysis was undertaken, focusing on three major players in this pathway: the genes coding for tryptophan hydroxylase 2 (neuronal TPH, TPH2), kynurenine-3-monooxygenase (K3MO), and kynurenine aminotransferase III (KATIII). Among the three different human KAT enzymes, KATIII is the enzyme expressed abundantly in neuroendocrine tissue and considered to be responsible for the mammalian brain KYNA production. KATIII shares up to 64.8% of its amino acid sequence with KATI and 30.1% with KATII, and can take over the function of these other enzymes.

So far, the role of the K3MO and KATIII genes in the vulnerability for major depression has not been described. The TPH2 gene, however, has already been studied in this context, as it codes for the rate limiting enzyme in the synthesis of serotonin. In 2005, a G-to-A transition at nucleotide 1463 of the TPH2 gene was discovered, replacing the highly conserved arginine at position 441 with histidine (R441H), and this rare mutation was found to be more frequent in MDD patients compared to controls. This finding, however, was not confirmed in subsequent studies. More systematic studies of the TPH2 gene looking at several SNPs and haplotypes did find some evidence for association with MDD.

In order to assess the potential role of the above three genes, SNP maps covering the genes were constructed, and single SNP and haplotype association analyses were performed in a sample of 338 German patients with a major depressive episode and 310 control individuals matched for age, ethnicity and gender.

### Experimental procedures:

*Test subjects.* The healthy control individuals (n=310) were recruited from two sites. 203 healthy control individuals were recruited at the Department of Psychiatry, University of Muenster, Germany. Another sample of 107 healthy control individuals from Antwerp University, Department of Neurology, were added in order to obtain age matching with the patient sample. All control individuals were from Caucasian origin. The total sample had a mean age of 48.52 years (± 14.11, f=178, m=132). The age and gender distribution did not differ significantly between patients and healthy control individuals.

*Single nucleotide polymorphism (SNP) selection.* SNPs were selected using the HAPMAP database (www.hapmap.org). Haplotype tagging SNPs necessary to identify all haplotypes circulating in the Caucasian population with a frequency of 2% or more were selected for genotyping. An area of 5 kb at both sides of the gene (according to HAPMAP) was included in the analysis. This resulted in the selection of 24 SNPs: 10 in K3MO, 8 in TPH2 and 6 in KATIII. The selected SNPs are shown in Table 1.

*SNP genotyping.* This was performed using the Sequenom MassARRAY iPLEX Gold® technology, following the protocol provided by Sequenom (www.sequenom.com). Polymerase chain reaction (PCR) and extension primers were designed using the Assay Design 3.1 program (Sequenom). Analysis and scoring were performed using the program Typer 3.4 (Sequenom).

*Genetic association analysis.* The PLINK package was used for single SNP and multipoint analyses. The threshold for the minor allele frequency was set at 0.01, the maximum SNP missingness rate at 0.5, the maximal individual missingness rate at 0.5, and the Hardy-Weinberg equilibrium (HWE) significance threshold at 0.001. The PLINK package was used as well to estimate which individuals carried the KATIII risk haplotype, using the "haplotype phases" module. The HAPMAP database was used to estimate the haplotype block structures of the three genes.

### Example 1:

### Genotyping results and HWE assessment

For K3MO, 28 of 649 individuals (23 patients, 5 control persons) had to be removed because of low genotyping success (>50% missing data). One marker (rs3014569) had to be removed because of exceeding the significance threshold in deviating from HWE.
For TPH2, 36 of 649 individuals (7 patients, 29 control persons) had to be removed because of low genotyping success. No markers had to be removed because of exceeding the significance threshold in deviating from HWE.

For KATIII finally, 27 of 649 individuals (22 patients, 5 control persons) had to be removed because of low genotyping success. No markers had to be removed because of exceeding the significance threshold in deviating from HWE.

### Example 2:

### Single SNP association results

The results of the single SNP association results are shown in Table 1. SNP rs1053230 in the K3MO gene showed borderline significant evidence for association, but not sufficient to withstand correction for multiple testing. No other SNP in the K3MO gene, and no SNP in TPH2 or KATIII showed evidence for association at this level.

**Table 1: SNP data and single SNP association results**

| Gene | SNP | Loc | A1 | A1 (PAT) | A1 (CON) | A2 | CHISQ | P |
|---|---|---|---|---|---|---|---|---|
| K3MO | rs2992642 | 238025973 | G | 0.247 | 0.258 | T | 0.201 | 0.654 |
| | rs10926513 | 238032286 | T | 0.415 | 0.403 | A | 0.174 | 0.677 |
| | rs6661244 | 238035345 | T | 0.354 | 0.321 | C | 1.250 | 0.264 |
| | rs6681337 | 238040707 | C | 0.087 | 0.090 | T | 0.031 | 0.859 |
| | rs3007737 | 238046600 | A | 0.436 | 0.398 | G | 1.587 | 0.208 |
| | rs3007736 | 238046871 | C | 0.357 | 0.334 | T | 0.710 | 0.399 |
| | rs602627 | 238070394 | G | 0.460 | 0.443 | A | 0.348 | 0.556 |
| | rs850678 | 238073180 | T | 0.192 | 0.182 | A | 0.194 | 0.660 |
| | rs1053230 | 238081389 | A | 0.200 | 0.249 | G | 4.068 | **0.044** |
| TPH2 | rs10748185 | 70622122 | G | 0.523 | 0.468 | A | 3.653 | 0.056 |
| | rs17110477 | 70631000 | A | 0.268 | 0.247 | T | 0.649 | 0.420 |
| | rs6582072 | 70640744 | A | 0.163 | 0.158 | G | 0.041 | 0.839 |
| | rs2171363 | 70646531 | T | 0.412 | 0.393 | C | 0.423 | 0.515 |
| | rs1386492 | 70648532 | G | 0.172 | 0.169 | A | 0.015 | 0.902 |
| | rs9325202 | 70693744 | A | 0.398 | 0.363 | G | 1.590 | 0.207 |
| | rs17110690 | 70694264 | A | 0.238 | 0.210 | G | 1.379 | 0.240 |
| | rs1386486 | 70698487 | T | 0.346 | 0.345 | C | 0.001 | 0.979 |
| KATIII | rs12729558 | 89114981 | G | 0.445 | 0.472 | C | 0.843 | 0.358 |
| | rs17130657 | 89115031 | G | 0.128 | 0.112 | C | 0.744 | 0.388 |
| | rs1325924 | 89132336 | C | 0.506 | 0.475 | T | 1.020 | 0.313 |
| | rs2038905 | 89135644 | A | 0.378 | 0.366 | T | 0.211 | 0.646 |
| | rs7556189 | 89150619 | C | 0.498 | 0.475 | G | 0.659 | 0.417 |
| | rs7517036 | 89155988 | T | 0.497 | 0.473 | A | 0.666 | 0.415 |

| | | | | | | | | |
|---|---|---|---|---|---|---|---|---|
| Loc: exact location on the chromosome in basepairs; A1: minor allele; A1 (PAT): Frequency of the A1 allele in patients with a major depressive episode; A1 (CON): Frequency of the A1 allele in control individuals; A2: major allele | | | | | | | | |

### Example 3:

### Haplotype analysis

For each gene, a haplotype analysis was performed using a sliding window approach, but also taking into account the haplotype block structure of the gene.

According to HAPMAP, the haplotype block structure of the K3MO gene is complex (data not shown). A small block is formed by SNPs rs10926513 and rs6661244. A two-point haplotype analysis of these SNPs showed no evidence for association. A larger block extends from SNP rs3007737 to rs1053230, comprising 5 SNPs. A sliding window analysis of this haplotype block using 2, 3, 4 and 5 SNP windows yielded no evidence for association.

Looking at the TPH2 gene, HAPMAP shows three consecutive blocks that are in loose linkage disequilibrium (LD) with each other. The first block extends from rs10748185 to rs17110477, the second from rs6582072 to rs1386492 and the last from rs9325202 to rs17110690. Each block was investigated with a sliding window analysis of 2 and 3 SNPs per window. No significant associations were detected.

Finally, the LD structure of KATIII consists of one single haplotype block according to HAPMAP (Figure 1). Sliding window analyses were performed using 2, 3, 4, 5 and 6 SNP windows applying PLINK. For all windows which include the first SNP (rs12729558), the overall haplotype distribution (OMNIBUS) was significantly different between patients with a major depressive episode and control individuals for all windows, with p-values ranging between 1.75 x 10⁻⁵ and 0.006 (Table 2). This is due to the haplotype CGCTCT (referring to the 6 SNP window analysis), which is found in about 5.7% of patients and 1.9% of healthy control persons. The bipolar (n=72) and MDD samples (n=266) were analyzed separately, again applying PLINK. In bipolar patients, the CGCTCT haplotype had a frequency of 7.1 %; significantly different from the frequency of 1.9% in the control population (p<0.001). In the MDD sample, the risk haplotype had a frequency of 5.3%, again significantly different from the control population (p<0.001).

**Table 2: Results of the sliding window haplotype analysis of KATIII for all windows which include marker rs12729558.**

| Window | SNPs | HAPLOTYPE | F_A | F_U | CHISQ | DF | P |
|---|---|---|---|---|---|---|---|
| 2SNP | rs12729558 | OMNIBUS | | | 29.510 | 3 | **<0.0001** |
| | rs17130657 | GG | 0.064 | 0.056 | 0.246 | 1 | 0.620 |
| | | CG | 0.059 | 0.000 | 28.950 | 1 | **<0.0001** |
| | | GC | 0.388 | 0.418 | 1.013 | 1 | 0.314 |
| | | CC | 0.490 | 0.525 | 1.317 | 1 | 0.251 |
| 3SNP | rs12729558 | OMNIBUS | | | 14.830 | 3 | **0.002** |
| | rs 17130657 | GGC | 0.068 | 0.090 | 2.038 | 1 | 0.153 |
| | rs1325924 | CGC | 0.057 | 0.018 | 12.880 | 1 | **0.0003** |
| | | GCC | 0.378 | 0.367 | 0.152 | 1 | 0.697 |
| | | CCT | 0.497 | 0.525 | 0.960 | 1 | 0.327 |
| 4SNP | rs12729558 | OMNIBUS | | | 14.860 | 3 | **0.002** |
| | rs17130657 | GCCA | 0.381 | 0.368 | 0.210 | 1 | 0.647 |
| | rs1325924 | GGCT | 0.065 | 0.089 | 2.392 | 1 | 0.122 |
| | rs2038905 | CGCT | 0.058 | 0.019 | 12.530 | 1 | **0.0004** |
| | | CCTT | 0.497 | 0.525 | 0.960 | 1 | 0.327 |
| 5SNP | rs12729558 | OMNIBUS | | | 14.270 | 3 | **0.003** |
| | rs17130657 | GCCAC | 0.380 | 0.366 | 0.267 | 1 | 0.606 |
| | rs1325924 | GGCTC | 0.064 | 0.090 | 3.040 | 1 | 0.081 |
| | rs2038905 | CGCTC | 0.056 | 0.020 | 11.370 | 1 | **0.0007** |
| | rs7556189 | CCTTG | 0.500 | 0.525 | 0.751 | 1 | 0.386 |
| 6SNP | rs12729558 | OMNIBUS | | | 14.620 | 4 | **0.006** |
| | rs17130657 | GCCACT | 0.368 | 0.357 | 0.143 | 1 | 0.706 |
| | rs1325924 | GGCTCT | 0.064 | 0.089 | 2.595 | 1 | 0.107 |
| | rs2038905 | CGCTCT | 0.057 | 0.019 | 12.210 | 1 | **0.0005** |
| | rs7556189 | GCCACA | 0.012 | 0.011 | 0.012 | 1 | 0.914 |
| | rs7517036 | CCTTGA | 0.500 | 0.525 | 0.756 | 1 | 0.385 |

| | | | | | | | |
|---|---|---|---|---|---|---|---|
| F_A: Frequency of the haplotype in patients; F_U: Frequency of the haplotype in healthy individuals | | | | | | | |

The frequency of the CGCTCT haplotype in a normal Caucasian population is estimated at 0% according to HAPMAP. The frequencies of the other haplotypes estimated by HAPMAP correlate quite well with the findings in the control population (Table 3).

**Table 3: KATIII haplotype frequencies in this study compared to the HAPMAP data. Data are shown for the full sample, and for the bipolar and MDD samples separately. The haplotype CGCTCT which drives the association (indicated in bold), is not present in the HAPMAP population.**

| | patients with MDE | Bipolar patients | MDD patients | healthy controls | Hapmap population |
|---|---|---|---|---|---|
| | (n=338) | (n=72) | (n=266) | | |
| CCTTGA | 0.500 | 0.554 | 0.484 | 0.525 | 0.467 |
| GCCACT | 0.368 | 0.307 | 0.385 | 0.357 | 0.375 |
| GGCTCT | 0.064 | 0.052 | 0.068 | 0.089 | 0.083 |
| GCCACA | 0.012 | 0.016 | 0.010 | 0.011 | 0.033 |
| CCTTCA | 0.000 | 0.000 | 0.000 | 0.000 | 0.017 |
| **CGCTCT** | **0.057** | **0.071** | **0.053** | **0.019** | **0.000** |

Next, the "haplotype phases" module of the PLINK package was used to identify carriers of the CGCTCT risk haplotype. This module reconstructs the haplotypes for each individual in the study. The module generated individual haplotypes for 617 individuals. In cases where two possible solutions were given (n=62), the solution with the highest posterior probability was selected. 34 out of 317 patients (10.7%) were found to be heterozygous carriers of the risk haplotype, while this was the case for none of the healthy control individuals. No homozygous carriers of the risk haplotype were found.

Nine out of 34 patients (26.5%) with the risk haplotype suffered from bipolar disorder (BD), and 25 had MDD, compared to the total sample of 72 BD patients on a total of 338 subjects (21.3%). This is not significantly different (Fischer's exact test p = 0.51). In the MDD patient group, 9 out of 25 haplotype carriers (36%) were diagnosed with melancholic features, compared to 79 out of 240 non-carrier MDD patients (33%). This again was not significantly different (Fischer's exact test p=0.82).

For exploratory reasons, the clinical data of the 34 patients carrying the risk haplotype were compared with the patients without risk haplotype (N = 304). Initially, a qualitative analysis of the risk patients' medical records was performed with focus on potentially distinctive features in depressive symptomatology, comorbidity and medication/treatment response. A tendency towards an elevated anxiety-level or even manifest panic disorder as well as an elevated agitation-level were isolated as noticeable elements of psychopathology. Then, a post hoc statistical analysis of numerical clinical data was performed to test the hypotheses. Numerical clinical data consisted of demographical information (age, gender, marital status etc.), DSM-IV diagnoses, HRDS-, YMRS-, BDI-, GAF- and CGI-ratings on inpatient admission and release and HRDS-/YMRS-ratings for each week during inpatient treatment for both the risk and non-risk patients with a major depressive episode. Non-parametrical correlations with risk-haplotype were significant only for HRDS-subscales *anxiety (psychological)* (ñ = 0.158, *p* = 0.01), *anxiety somatic* (ñ = 0.191, *p* = 0.01) and *genital symptoms* (ñ = 0.142, *p* = 0.05) at the time of admission. There was no significant correlation between risk-haplotype and other psychometric subscales, for example those indicating an elevated agitation-level. The correlation of HRDS-subscale *genital symptoms* with risk-haplotype remained significant by the time of release (ñ = .142, *p* = 0.05). A group comparison by Mann-Whitney-U-Test between the risk and non-risk patients yielded the same results: Group differences were significant only for HRDS-subscales *anxiety (psychological)* (U = 2623.000, *p* = 0.007), *anxiety somatic* (U = 2395.000, *p* = 0.001) and *genital symptoms* (U = 2785.000, *p* = 0.015) at the time of admission and remained significant for *genital symptoms* by the time of release (U = 2674.500, *p* = 0.015). Reported statistical analyses were not corrected for multiple testing because of their exploratory character.

### Discussion:

Immune dysfunction in major depression is well established, and the kynurenine pathway is crucial in this respect. However, a systematic screening of genes related to this pathway with regard to the genetic vulnerability for major depression has been lacking. Here it was attempted to partially bridge this gap. While only three genes were examined, the approach was thorough, in the sense that each of these genes was covered by haplotype tagging SNPs reaching beyond both ends of the gene.

Regarding the TPH2 gene, the results of the study were fully negative, both at the single SNP and at the haplotype level. TPH2 has been studied before with regard to MDD, but after an initial promising report, studies were ambiguous. This study confers an unequivocal negative finding to this field.

For K3MO, no genetic studies with affective disorder patients have been published until now. A single SNP (rs1053230) yielded a borderline significant result. However, this finding would disappear with correction for multiple tests, and the haplotype analysis was completely negative. The conclusion is that this study does not support a potential role for the K3MO gene in the vulnerability for affective disorder.

The analysis of the KATIII genotype data yielded more promising results. The haplotype analysis showed significant results for all haplotype windows containing. SNP rs12729558. Further exploration made clear that this is due to the fact that a specific haplotype (CGCTCT) had an estimated frequency of 6% in patients, compared to 2% in controls. When reconstructing individual haplotypes with the best possible posterior probability, 10.7% of MDD patients were estimated to carry this risk haplotype, while none of the control persons did. The significance of these findings is strong enough to withstand any correction for multiple testing.

The potential importance of KATIII in major depression pathogenesis is evident. Kynurenic acid (KYNA), a metabolite of tryptophan, is a broad-spectrum antagonist of excitatory amino acid receptors, suggesting it may participate in normal brain function as a modulator of glutamatergic neurotransmission. The level of KYNA is altered in several neurodegenerative diseases. KATIII is a kynurenine aminotransferase that forms KYNA by transamination of kynurenine. Although KATII is the primary enzyme that forms KYNA from kynurenine in the brain, KATII -/- knock-out mice did not show change in KYNA level in the brain. KATIII is one of the kynurenine aminotransferases that forms KYNA by transamination of kynurenine and present abundantly in the neuroendocrine tissues. Moreover, it shares part of the structures of both mammalian KATI and KATII and is therefore thought to take over KATII function in case of KATII -/- knock-out mice. It was shown that the activity of KATIII on forming KYNA from kynurenine is reduced in the presence of 30HK. The activity of KATIII in the presence of immune activation where there would be an associated increase formation of 30HK would be of importance in circumstances of significantly low KYNA demonstrated in major depression. The particular haplotype demonstrated here could have a particular functional relationship in this regard.

Comparing the clinical features, haplotype carriers seem to show more anxiety related symptoms compared to non-carriers, with significantly higher scores on the HDRS items "anxiety (psychological)", "anxiety somatic" and "genital symptoms". It was examined whether the higher occurrence of "genital symptoms" could be related to differences in antidepressant medication intake between both groups, but this seemed not to be the case: both groups do not differ regarding the "overall" antidepressant drug intake on admission or regarding the type of antidepressant they received at the end of treatment. Suffering from MDD or bipolar disorder appears not to be relevant regarding KATIII genotype.

In summary, while it is not clear how this haplotype would affect KATIII function and KYNA production, an interesting risk haplotype for major depression with anxiety symptoms has been identified.

## Claims

1. Diagnostic marker comprising at least one single nucleotide polymorphism (SNP) in the human KATIII gene.

2. Diagnostic marker according to claim 1, comprising the SNP rs12729558.

3. Diagnostic marker according to claim 2, further comprising at least one further SNP selected from the group consisting of SNPs rs17130657, rs1325924, rs2038905, rs7556189, rs7517036 and combinations thereof.

4. Diagnostic marker according to claim 3, consisting of SNPs rs12729558, rs17130657, rs1325924, rs2038905, rs7556189, and rs7517036.

5. Diagnostic marker according to any one of claims 1 to 4, being indicative of a predisposition for a psychiatric disorder or a psychiatric disease.

6. Diagnostic marker according to claim 5, wherein
the haplotype C in SNP rs12729558; or
the haplotype C in SNP rs12729558 and the haplotype G in SNP rs17130657; or
the haplotype C in SNP rs12729558 and the haplotype G in SNP rs17130657 and the haplotype C in SNP rs1325924; or
the haplotype C in SNP rs12729558 and the haplotype G in SNP rs17130657 and the haplotype C in SNP rs1325924 and the haplotype T in SNP rs2038905; or
the haplotype C in SNP rs12729558 and the haplotype G in SNP rs17130657 and the haplotype, C in SNP rs1325924 and the haplotype T in SNP rs2038905 and the haplotype C in SNP rs7556189; or
the haplotype C in SNP rs12729558 and the haplotype G in SNP rs17130657 and the haplotype C in SNP rs1325924 and the haplotype T in SNP rs2038905 and the haplotype C in SNP rs7556189 and the haplotype T in SNP rs7517036;
indicates a predisposition for a psychiatric disorder or a psychiatric disease.

7. Diagnostic marker according to claim 5 or claim 6, wherein the psychiatric disorder or the psychiatric disease is selected from the group consisting of mood disorders, bipolar disorder, major depressive disorder (MDD), and anxiety disorders.

8. A method for the detection of a predisposition for a psychiatric disorder or a psychiatric disease in a human patient, comprising the steps of:
(a) providing a genetic sample derived from the patient; and
(b) determining the haplotype of at least one single nucleotide polymorphism (SNP) in the patient's KATIII gene *in vitro.*

9. Method according to claim 9, wherein the at least one SNP comprises the SNP rs12729558.

10. Method according to claim 10, wherein further the haplotype of at least one further SNP selected from the group consisting of SNPs rs17130657, rs1325924, rs2038905, rs7556189, and rs7517036 is determined.

11. Method according to claim 11, wherein the haplotype of the SNPs rs12729558, rs17130657, rs1325924, rs2038905, rs7556189, and rs7517036 is determined.

12. Method according to any one of claims 9 to 12, further comprising the step of correlating
the haplotype C in SNP rs12729558; or
the haplotype C in SNP rs12729558 and the haplotype G in SNP rs17130657; or
the haplotype C in SNP rs12729558 and the haplotype G in SNP rs17130657 and the haplotype C in SNP rs1325924; or
the haplotype C in SNP rs12729558 and the haplotype G in SNP rs17130657 and the haplotype C in SNP rs1325924 and the haplotype T in SNP rs2038905; or
the haplotype C in SNP rs12729558 and the haplotype G in SNP rs17130657 and the haplotype C in SNP rs1325924 and the haplotype T in SNP rs2038905 and the haplotype C in SNP rs7556189; or
the haplotype C in SNP rs12729558 and the haplotype G in SNP rs17130657 and the haplotype C in SNP rs1325924 and the haplotype T in SNP rs2038905 and the haplotype C in SNP rs7556189 and the haplotype T in SNP rs7517036;
to a predisposition for a psychiatric disorder or a psychiatric disease.

13. Method according to any one of claims 9 to 13, wherein the psychiatric disorder or the psychiatric disease is selected from the group consisting of mood disorders, bipolar disorder, major depressive disorder (MDD), and anxiety disorders.

14. Use of at least one single nucleotide polymorphism (SNP) in the human KATIII gene as a diagnostic marker, wherein the diagnostic marker is indicative of a predisposition for a psychiatric disorder or a psychiatric disease.

15. Use according to claim 14, wherein the SNP is selected from the group, consisting of SNPs rs12729558, rs17130657, rs1325924, rs2038905, rs7556189, and rs7517036.
